# EUROPÄISCHE PATENTANMELDUNG

(11) **EP 1 252 926 A2**
(43) Veröffentlichungstag der Anmeldung: **30.10.2002**
(21) Anmeldenummer: 02007354.0
(22) Anmeldetag: 08.04.2002
(51) Int. Cl.: B01J 23/46, B01J 21/04, C07C 209/72

(54) **Ruthenium-Katalysator für die Hydrierung von Diaminodiphenylmethan zu Diaminocyclohexylmethan**

(30) Priorität: 19.04.2001 DE 10119136
(71) Anmelder: BAYER AG, 51368 Leverkusen (DE)
(72) Erfinder: Schulze Tilling, Andreas, Dr., League City, TX 77573 (US); Prinz, Thomas, Dr., 51371 Leverkusen (DE); Kintrup, Jürgen Dr., 51373 Leverkusen (DE)

(57) **Zusammenfassung**

Die Erfindung betrifft Ruthenium-Katalysatoren für die Hydrierung von Diaminodiphenylmethan (MDA) zu Diaminodicyclohexylmethan (PACM), insbesondere mit einem Anteil an trans,trans-4,4'-Diaminodicyclohexylmethan von 17 bis 24 %, in einem kontinuierlich betriebenen Suspensionsreaktor, wobei Ruthenium auf einen Träger aus hochreinem Aluminiumoxid aufgebracht ist, das vorzugsweise einen Gehalt an Natrium von weniger als 0,05 Gew.-%, eine Korngröße von 5 bis 150 µm und eine spezifische Oberfläche nach BET von 30 bis 300 m²/g aufweist.

## Beschreibung

Die vorliegende Erfindung betrifft Ruthenium-Katalysatoren für die Hydrierung von Diaminodiphenylmethan (MDA) zu Diaminodicyclohexylmethan (PACM) in einem kontinuierlich betriebenen Suspensionsreaktor, wobei Ruthenium auf einen Träger aus hochreinem Aluminiumoxid aufgebracht ist.

PACM wird technisch durch Hydrierung von MDA hergestellt. PACM findet beispielsweise Anwendung zur Herstellung von Lacken, hauptsächlich als Vorstufe für den Lackrohstoff Diisocyanatodicyclohexylmethan. Bei einer Reihe von Anwendungen ist das Isomerenverhältnis von besonderer Bedeutung.

Aus EP 639 403 A2 ist ein Katalysator zur Herstellung von PACM mit niedrigem Anteil an trans-trans-Isomer durch Hydrierung von MDA bekannt. Es handelt sich dabei um einen Katalysator mit einer dünnen Ruthenium- oder Rhodium-haltigen Schicht auf einem speziellen Träger, nämlich einer kalzinierten und oberflächlich rehydratisierten Übergangstonerde, insbesondere Hydrargillit oder Bayerrit

EP 639 403 A2 beschreibt die Desaktivierung des Katalysators durch höhermolekulare Bestandteile des Reaktionsgemischs und die Einstellung eines niedrigen Anteils an trans,trans-Isomerem im Produkt als Problem bei der technischen Herstellung von PACM. Durch Verwendung des speziellen Katalysators sollen diese Probleme gelöst werden. Der spezielle Katalysator eignet sich aber hauptsächlich für einen Einsatz in Reaktoren mit einem Katalysatorfestbett, in denen der Katalysator während des Betriebes nicht ausgetauscht werden kann. Zudem wird ein großer Teil des Reaktorvolumens durch den inaktiven Kern des verwendeten Schalenkatalysators ausgefüllt und steht als Reaktionsvolumen nicht mehr zur Verfügung.

Die Durchführung von Hydrierungen in diskontinuierlich betriebenen Suspensionsreaktoren ist bereits beschrieben. Suspensionsreaktoren haben den Vorteil, dass der verbrauchte Katalysator leicht ausgewechselt werden kann.

Aufgabe der Erfindung war es daher, einen Katalysator zur Verfügung zu stellen, der die Hydrierung von MDA zu PACM, insbesondere mit einem geringen Anteil an trans,trans-4,4'-Diaminodicyclohexylmethan, in einem kontinuierlich betriebenen Suspensionsreaktor mit hoher Raum-Zeit-Ausbeute und einer hohen Katalysatorlebensdauer erlaubt.

Überraschend wurde gefunden, dass man bei Verwendung von hochreinem Aluminiumoxid als Träger pulverförmige, suspendierbare Rutheniumkatalysatoren herstellen kann, mit denen MDA mit hoher Raum-Zeit-Ausbeute zu PACM mit einem geringen Anteil an trans,trans-4,4'-Diaminodicyclohexylmethan bei hoher Katalysatorlebensdauer hydriert werden kann.

Gegenstand der Erfindung sind Ruthenium-Katalysatoren für die Hydrierung von Diaminodiphenylmethan (MDA) zu Diaminodicyclohexylmethan (PACM) in einem kontinuierlich betriebenen Suspensionsreaktor, die dadurch gekennzeichnet sind, dass Ruthenium auf einen Träger aus hochreinem Aluminiumoxid aufgebracht ist.

Das als Trägermaterial verwendete Aluminiumoxid zeichnet sich insbesondere dadurch aus, dass es nur geringe Mengen an Alkalimetallen, insbesondere Natrium, enthält.

Dass Katalysatoren auf einem hochreinen Träger, insbesondere mit einem besonders niedrigen Gehalt an Natrium als Hydrierkatalysatoren vorteilhaft sind, überrascht, da in anderen Fällen gerade Alkaliverbindungen als Promotoren zugesetzt werden. So beschreibt WO 9608462 A1 ein Verfahren zur katalytischen Hydrierung von aromatischen Aminen in Gegenwart eines Edelmetallkatalysators und Lithiumhydroxid als Promotor.

Vorzugsweise weist das hochreine Aluminiumoxid, das erfindungsgemäß als Trägermaterial eingesetzt wird, einen Gehalt an Natrium von weniger als 0,05 Gew.-%, besonders bevorzugt von höchstens 0,02 Gew.-%, berechnet als Na₂O auf.

Solche hochreinen Aluminiumoxide werden technisch beispielsweise durch die Hydrolyse von Aluminiumalkoholaten hergestellt. Aluminiumoxide, die bisher üblicherweise als Trägermaterialien für Hydrierkatalysatoren eingesetzt wurden, haben einen Gehalt an Na₂O im Bereich von 0,1 bis 0,5 Gew.-%.

Die aus EP 639 403 A2 bekannten speziellen Träger für Hydrierkatalysatoren, nämlich kalzinierte und oberflächlich rehydratisierte Übergangstonerde, insbesondere Hydrargillit oder Bayerit basieren ebenfalls auf Aluminiumoxid. Gemäß EP 639 403 A2 sind jedoch nur solche Träger für Ruthenium-haltige Hydrierkatalysatoren vorteilhaft, die eine bestimmte basische Pufferkapazität aufweisen. Die Pufferkapazität der erfindungsgemäßen Aluminiumoxide liegt nicht in dem als vorteilhaft angesehenen Bereich. Über die Vorteile eines Aluminiumoxid-Trägers mit geringem Gehalt an Natrium ist in EP 639 403 A2 nichts gesagt. Zudem wird in EP 639 403 A2 insbesondere für Festbettkatalysatoren aufgezeigt, dass eine schalenförmige Verteilung des Rutheniums auf dem Katalysatorträger vorteilhaft ist. Im Gegensatz dazu ist es bei den erfindungsgemäßen Katalysatoren, insbesondere bei deren Einsatz als Pulver in einem Suspensionsreaktor besonders vorteilhaft, wenn das Ruthenium über den gesamten Querschnitt der Trägerpartikel verteilt ist. Dies führt zu einer besseren Aktivität und Standzeit des Katalysators.

Bevorzugt ist bei den erfindungsgemäßen Ruthenium-Katalysatoren das Ruthenium daher über den gesamten Querschnitt der Trägerpartikel verteilt.

Bei dem als Trägermaterial eingesetzten hochreinen Aluminiumoxid handelt es sich vorzugsweise um ein Pulver mit einem mittleren Korndurchmesser von 5 bis 150 µm, besonders bevorzugt von 10 bis 120 µm, insbesondere bevorzugt von 30 bis 100 µm.

Vorzugsweise weist das eingesetzte hochreine Aluminiumoxid eine spezifische Oberfläche nach BET von 30 bis 300 m²/g, besonders bevorzugt von 70 bis 200 m²/g, insbesondere bevorzugt von 100 bis 160 m²/g auf.

Das Porenvolumen des hochreinen Aluminiumoxids beträgt bei Berücksichtigung von Poren mit einem Durchmesser <10000 nm bevorzugt 0,1 bis 1,5 ml/g, besonders bevorzugt 0,3 bis 0,7 ml/g.

Neben hochreinem Aluminiumoxid können als Trägermaterialien auch Aluminiumhaltige, alkaliarme Mischoxide eingesetzt werden, die die gleichen physikalischen Eigenschaften aufweisen wie hochreines Aluminiumoxid.

Vorzugsweise liegen die erfindungsgemäßen Ruthenium-Katalysatoren in Pulverform vor.

Der Gehalt an Ruthenium beträgt vorzugsweise 1 bis 10 Gew.-%, besonders bevorzugt 4 bis 8 Gew.-%.

Die erfindungsgemäßen Ruthenium-Katalysatoren können neben Ruthenium auch andere Metalle, beispielsweise Rhodium enthalten.

Vorzugsweise zeichnen sich die Ruthenium-Katalysatoren durch gute Filtrierbarkeit aus und dadurch, dass der Katalysator nach seinem Einsatz und einer Abtrennung der Produktlösung erneut für die Hydrierung von Diaminodiphenylmethan (MDA) zu Diaminodicyclohexylmethan (PACM) in einem kontinuierlich betriebenen Suspensionsreaktor eingesetzt werden kann.

Bevorzugt sind Ruthenium-Katalysatoren, die als Katalysatoren für die Hydrierung von Diaminodiphenylmethan (MDA), das neben MDA höhermolekulare aromatische Amine enthält, zu Diaminodicyclohexylmethan (PACM) geeignet sind.

Die erfindungsgemäßen Katalysatoren lassen sich beispielsweise herstellen, indem man den erfindungsgemäßen hochreinen Aluminiumoxid-Träger in Wasser suspendiert und anschließend eine wässrige Lösung einer Rutheniumverbindung oder eines Rutheniumsalzes, wie z.B. Rutheniumchlorid oder Rutheniumnitrosylnitrat zusetzt. Man läßt das Ruthenium auf dem Träger adsorbieren und setzt anschließend eine Base, z.B. Natriumcarbonat, Natronlauge oder Lithiumhydroxid zur Fällung des Rutheniums zu. Anschließend kann ein Reduktionsmittel, z.B. Formaldehyd, Natriumformiat oder Hydrazin zugesetzt werden. Anschließend wird filtriert, der Katalysator Chlorid- und Natrium-frei gewaschen und getrocknet. Der getrocknete pulverförmige Katalysator kann zusätzlich mit Wasserstoff bei Temperaturen von 100 bis 250°C in einem Reduktionsofen reduziert und mit Inertgas/Luft-Mischung passiviert werden. Der Katalysator kann aber auch in einem Hydrierreaktor, in dem die Hydrierung von MDA zu PACM erfolgen soll, in einem Lösungsmittel suspendiert und dort mit Wasserstoff reduziert werden.

Die erfindungsgemäßen Ruthenium-Katalysatoren werden zur Hydrierung von Diaminodiphenylmethan (MDA) zu Diaminodicyclohexylmethan (PACM) in einem kontinuierlich betriebenen Suspensionsreaktor eingesetzt.

Vorzugsweise werden die Ruthenium-Katalysatoren zur Herstellung von Diaminodicyclohexylmethan (PACM) mit einem Anteil an trans,trans-4,4'-Diaminodicyclohexylmethan von 17 bis 24 % verwendet.

Der Einsatz der erfindungsgemäßen Ruthenium-Katalysatoren zur Hydrierung von Diaminodiphenylmethan (MDA) zu Diaminodicyclohexylmethan (PACM) in einem kontinuierlich betriebenen Suspensionsreaktor erfolgt beispielsweise bei einem Wasserstoffdruck von 50 bis 400 bar, bevorzugt von 100 bis 200 bar.

Wasserstoff wird vorteilhaft in einem Überschuss von 5 bis 200 %, bevorzugt von 20 bis 100 % der Theorie zugegeben.

Die Temperatur beträgt beispielsweise von 130 bis 190°C, bevorzugt von 150 bis 180°C.

Der erfindungsgemäße Katalysator kann beispielsweise in einer Menge von 1 bis 10 Gew.-%, bevorzugt 3 bis 8 Gew.-%, bezogen auf die Reaktionsmischung eingesetzt werden.

Mittels der Parameter Katalysatorkonzentration, Temperatur und Verweilzeit im Reaktor kann der Gehalt an trans,trans-Isomer im Produkt eingestellt werden. So sind Produkte mit einem niedrigen Anteil an trans,trans-Isomer, insbesondere mit einem Anteil zwischen 17 und 24 % zu erreichen. Beispielsweise kann bei gegebener Temperatur und Katalysatorkonzentration durch Anpassen der Verweilzeit der Reaktionsmischung im Reaktor der Anteil an trans,trans-Isomer im Produkt eingestellt werden.

Die Hydrierung erfolgt in einem Suspensionsreaktor, vorzugsweise einem Rührkessel oder einer Blasensäule, besonders bevorzugt in einer Kaskade mehrerer hintereinandergeschalteter Rührkessel oder Blasensäulen.

Die Vermischung von Edukt (MDA) mit dem eingesetzten Katalysator und dem zur Hydrierung benötigten Wasserstoff erfolgt bei Verwendung von Rührkesseln als Suspensionsreaktoren mittels eines Rührers, bei Verwendung von Blasensäulen als Suspensionsreaktoren durch den Eintrag von Wasserstoff mit hoher Geschwindigkeit und die Erzeugung einer turbulenten Strömung im Reaktor.

Die Hydrierung kann mit und ohne den Zusatz von organischen Lösungsmitteln durchgeführt werden. Als Lösungsmittel eignen sich beispielsweise Alkohole, bevorzugt sekundäre Alkohole, z.B. Isobutanol, Cyclohexanol oder Methylcyclohexanol oder tertiäre Alkohole, z.B. tert.-Butanol, besonders bevorzugt tertiäre Alkohole.

Vorzugsweise wird so vorgegangen, dass der Katalysator zusammen mit dem Reaktionsgemisch durch den Suspensionsreaktor gefördert wird. Anschließend erfolgt die Kühlung der Produktmischung, die Abscheidung überschüssigen Wasserstoffs und eine Filtration des Katalysators. Das gegebenenfalls eingesetzte Lösungsmittel kann nach Abtrennung des Katalysators destillativ vom Produkt getrennt und in den Hydrierprozess zurückgeführt werden.

Erfindungsgemäße Katalysatoren zeichnen sich auch nach längerem Einsatz durch gute Filtrierbarkeit und hohe mechanische Stabilität aus.

Vorzugsweise wird der Katalysator nach der Abtrennung der Produktlösung erneut zur Hydrierung von MDA eingesetzt.

Es ist bezüglich der Katalysatoraktivität und der Lebensdauer vorteilhaft, den Katalysator nach der Abtrennung der Produktlösung mit einem inerten Lösungsmittel zu waschen. Hierdurch kann die Katalysatoroberfläche von Ablagerungen höhermolekularer Reaktionsprodukte befreit werden.

Bei abfallender Katalysatoraktivität nach längerem Betrieb können Teilmengen des Katalysators ausgeschleust und durch frischen Katalysator ersetzt werden, so dass eine Anlage zur Durchführung des erfindungsgemäßen Verfahrens mit konstanter mittlerer Katalysatoraktivität und konstantem Durchsatz betrieben werden kann.

Im Folgenden wird die Erfindung anhand von Beispielen weiter erläutert. Die Beispiele stellen einzelne Ausführungsformen der Erfindung dar, die Erfindung ist jedoch nicht auf die Beispiele beschränkt.

### Beispiel 1 Katalysatorherstellung

In einem 10 1 Reaktionsgefäß wurden 855 g Aluminiumoxid (Al₂O₃) mit einer spezifischen Oberfläche nach BET von 160 m²/g, einem mittleren Korndurchmesser von 50 µm und einem Natriumgehalt von 0,02 Gew.-%, berechnet als Na₂O, unter Rühren in 3600 ml vollentsalztem Wasser suspendiert. Anschließend wurde 10 Minuten gerührt. In die Suspension wurden unter Rühren 360 ml einer Lösung bestehend aus 225 g Rutheniumchlorid-Lösung mit 20 Gew.-% Ruthenium-Gehalt (entspricht 45 g Ruthenium) und vollentsalztem Wasser gegeben. Danach ließ man 1 Stunde nachrühren. In die Suspension pumpte man unter Rühren in 40 Minuten ca. 695 g 10 gew.-%ige Natriumhydroxid-Lösung bis sich ein pH-Wert von 8 eingestellt hatte. Danach ließ man 1 Stunde nachrühren und stellte abermals mit 10 gew.-%iger Natriumhydroxid-Lösung auf pH 8 ein. Anschließend wurde eine Lösung von 200 g Hydrazinhydrat in 4 1 Wasser zugesetzt und eine weitere Stunde gerührt. Die entstandene Katalysatorsuspension wurde filtriert und so lange mit vollentsalztem Wasser gewaschen, bis das ablaufende Wasser neutral und chloridfrei war. Der Filterkuchen wurde 30 Minuten trockengesaugt. Abschließend wurde der Katalysator im Vakuumtrockenschrank bei 110°C getrocknet.

### Beispiel 2 Katalysatortestung in einem kontinuierlich betriebenen Rührkessel

MDA wurde in einem kontinuierlich betriebenen Rührkessel hydriert. Das Reaktionsvolumen betrug 330 ml; es wurde ein gemäß Beispiel 1 hergestellter, pulverförmiger Katalysator in einer Katalysatorkonzentration von 5 Gew.-% in dem Rührkessel vorgelegt. MDA wurde in technischer Qualität (Bezeichnung MDA 90/10) mit einem Anteil von ca. 10 % an höhermolekularen Komponenten als 33 gew.-%ige Lösung in Isobutanol eingesetzt. Das MDA 90/10-Isobutanol-Gemisch wurde aus einem Vorlagenbehälter in den Reaktor dosiert. Der Reaktordruck wurde durch ständiges Nachführen von Wasserstoff auf 150 bar konstant gehalten. Bei dem Versuch wurde eine Temperatur von 150°C eingestellt.

Der Überlauf des Reaktionsgemisches erfolgte über einen weiteren Behälter, aus dem Proben zur Analyse gezogen wurden. Durch Variation der Förderleistung der Dosierpumpe wurden verschiedene mittlere Verweilzeiten eingestellt. Die Proben wurden mittels Gaschromatographie analysiert.

Die Gehalte an MDA, [H₆]-MDA (4-Aminocyclohexyl-4-aminophenylmethan), PACM und der Anteil an trans,trans-PACM (tt-Anteil) sind in Tabelle 1 wiedergegeben.

| | |
|---|---|
| Temperatur [°C] | 150 |
| Verweilzeit [min] | 61 |
| Durchsatz [g(PACM) pro 1 und h] | 247 |
| PACM [%] | 88 |
| tt-Anteil [%] | 24 |
| [H₆]-MDA [%] | 1,5 |
| MDA [%] | 0,1 |
| Höhermolekulare [%] | 10 |

### Beispiel 3 Katalysatortestung in einer kontinuierlich betriebenen Kaskade aus 3 Rührkesseln

Beispiel 2 wurde wiederholt, allerdings wurde die Verweilzeit so gekürzt, dass nur Teilumsatz erfolgte. Anschließend wurde das Produkt zwei weitere Male durch den Reaktor gefördert. Das Produkt entspricht dem Produkt, das in einer Kaskade aus drei Rührkesseln erhalten wird.

Die Gehalte an MDA, [H₆]-MDA, PACM und der Anteil an trans,trans-PACM (tt-Anteil) sind in Tabelle 2 wiedergegeben.

| | |
|---|---|
| Temperatur [°C] | 150 |
| Verweilzeit [min] | 45 |
| Durchsatz [g(PACM) pro 1 und h] | 330 |
| PACM [%] | 89 |
| tt-Anteil [%] | 19 |
| [H₆]-MDA [%] | 0,9 |
| MDA [%] | 0,1 |
| Höhermolekulare [%] | 10 |

Der Versuch zeigt, dass bei Einsatz einer Kaskade von drei Reaktoren gleichzeitig vollständiger Umsatz und ein trans,trans-Anteil im Bereich von 20 % erzielt werden kann und zugleich eine hohe Raum-Zeit-Ausbeute erreicht wird.

### Beispiel 4 Katalysatortestung in einem diskontinuierlich betriebenen Rührkessel

MDA wurde in einem diskontinuierlich betriebenen Rührkessel hydriert. Das Reaktionsvolumen betrug 330 ml; es wurde ein gemäß Beispiel 1 hergestellter, pulverförmiger Katalysator in einer Katalysatorkonzentration von 5 Gew.-% in den Rührkessel vorgelegt. MDA wurde in technischer Qualität (Bezeichnung MDA 90/10) mit einem Anteil von ca. 10 % an höhermolekularen Komponenten als 33 gew.-%ige Lösung in Isobutanol eingesetzt. 330 ml des MDA 90/10-Isobutanol-Gemischs wurden aus einem Vorlagenbehälter in den Reaktor dosiert. Der Reaktordruck wurde durch ständiges Nachführen von Wasserstoff auf 150 bar konstant gehalten. Bei dem Versuch wrude eine Temperatur von 150°C eingestellt.

Aus dem Reaktionsgemisch wurden nach unterschiedlicher Verweilzeit des Reaktionsgemischs im Reaktor Proben zur Analyse gezogen. Die gezogenen Proben wurden mittels Gaschromatographie analysiert.

Die Gehalte an MDA, [H₆]-MDA, PACM und der Anteil an trans,trans-PACM (tt-Anteil) sind in Tabelle 3 wiedergegeben.

| | | | |
|---|---|---|---|
| Temperatur [°C] | 150 | 150 | 150 |
| Verweilzeit [min] | 51 | 111 | 171 |
| Durchsatz [g(PACM) pro 1 und h] | 282 | 131 | 81 |
| PACM [%] | 84 | 85 | 81 |
| tt-Anteil [%] | 14 | 24 | 37 |
| [H₆]-MDA [%] | 3,1 | 0,2 | 0,3 |
| MDA [%] | 0,5 | 0,1 | 0 |
| Höhermolekulare [%] | 12 | 14 | 18 |

## Patentansprüche

1. Ruthenium-Katalysator für die Hydrierung von Diaminodiphenylmethan (MDA) zu Diaminodicyclohexylmethan (PACM) in einem kontinuierlich betriebenen Suspensionsreaktor, **dadurch gekennzeichnet, dass** Ruthenium auf einen Träger aus hochreinem Aluminiumoxid aufgebracht ist.

2. Ruthenium-Katalysator gemäß Anspruch 1, **dadurch gekennzeichnet, dass** das hochreine Aluminiumoxid einen Gehalt an Natrium von weniger als 0,05 Gew.-%, berechnet als Na₂O aufweist.

3. Ruthenium-Katalysator gemäß wenigstens einem der Ansprüche 1 bis 2, **dadurch gekennzeichnet, dass** es sich bei dem hochreinen Aluminiumoxid um ein Pulver mit einem mittleren Korndurchmesser von 5 bis 150 µm handelt.

4. Ruthenium-Katalysator gemäß wenigstens einem der Ansprüche 1 bis 3, **dadurch gekennzeichnet, dass** es sich bei dem hochreinen Aluminiumoxid um ein Pulver mit einer spezifischen Oberfläche nach BET von 30 bis 300 m²/g handelt.

5. Ruthenium-Katalysator gemäß wenigstens einem der Ansprüche 1 bis 4, **dadurch gekennzeichnet, dass** es sich dabei um ein Pulver handelt.

6. Ruthenium-Katalysator gemäß wenigstens einem der Ansprüche 1 bis 5, **dadurch gekennzeichnet, dass** der Gehalt an Ruthenium 1 bis 10 Gew.-% beträgt.

7. Ruthenium-Katalysator gemäß wenigstens einem der Ansprüche 1 bis 6, **dadurch gekennzeichnet, dass** das Ruthenium über den gesamten Querschnitt der Trägerpartikel verteilt ist.

8. Ruthenium-Katalysator gemäß wenigstens einem der Ansprüche 1 bis 7, **dadurch gekennzeichnet, dass** er gut filtrierbar ist.

9. Ruthenium-Katalysator gemäß wenigstens einem der Ansprüche 1 bis 8, **dadurch gekennzeichnet, dass** er nach seinem Einsatz und einer Abtrennung der Produktlösung erneut für die Hydrierung von Diaminodiphenylmethan (MDA) zu Diaminodicyclohexylmethan (PACM) in einem kontinuierlich betriebenen Suspensionsreaktor eingesetzt werden kann.

10. Ruthenium-Katalysator gemäß wenigstens einem der Ansprüche 1 bis 8, **dadurch gekennzeichnet, dass** er als Katalysator für die Hydrierung von Diaminodiphenylmethan (MDA), das neben MDA höhermolekulare aromatische Amine enthält, zu Diaminodicyclohexylmethan (PACM) geeignet ist.

11. Verwendung des Ruthenium-Katalysators gemäß wenigstens einem der Ansprüche 1 bis 10 zur Herstellung von Diaminodicyclohexylmethan (PACM) mit einem Anteil an trans,trans-4,4'-Diaminodicyclohexylmethan von 17 bis 24 %.
